# EUROPEAN PATENT SPECIFICATION

(11) **EP 2 914 323 B1**
(45) Date of publication and mention of the grant of the patent: **12.12.2018**
(21) Application number: 13850523.5
(22) Date of filing: 28.10.2013
(51) Int. Cl.: A61M 16/04, A61B 1/32, A61B 17/02, A61B 17/34, A61M 16/20, A61M 25/09, A61M 25/06

(54) **AN INTUMASK ASSEMBLY**
INTUMASK-ANORDNUNG
ENSEMBLE MASQUE À TUBE INTERNE

(30) Priority: 30.10.2012 US 201261719972 P
(43) Date of publication of application: 09.09.2015
(73) Proprietor: Breslauer Ltd., 49513 Petach Tikva (IL)
(72) Inventor: BRESLAUER, Haim, 49001 Petach Tikva (IL); BESHARIM, Shlomo, 8496500 Omer (IL); BESHARIM, Eli, 8480605 Be'er - Sheva (IL)
(74) Representative: Lecomte & Partners
(86) International application number: PCT/IL2013/050879
(87) International publication number: WO 2014/068558

(56) References cited:
- WO-A1-2008/000204
- GB-A- 2 317 830
- GB-B- 2 445 655
- US-A- 6 003 514
- US-A1- 2007 102 001
- US-A1- 2007 163 597

## Description

### FIELD OF THE INVENTION

This invention generally pertains to artificial airway assemblies. In particular, it relates to artificial airway assemblies in which the airway tube is designed for ease of function and placement, and for ensuring that the airway assembly is secured in the correct position.

### BACKGROUND OF THE INVENTION

US patent application 2003192548 discloses a laryngeal mask tube which consists of a dual-airway tube, a mask with an inflatable bladder and an inflation indicator device. The dual-airway tube comprises a simplified primary tube and secondary tube integrally combined. The primary tube communicates with the mask to guide gas into the body of a patient and the secondary tube communicates between with the bladder and the inflation indicator device to inflate the bladder and monitor the inflation. Moreover, two ribs are formed in the mask to prevent blockage of the primary tube, and a tongue is formed inside the bladder to prevent the bladder from folding and preventing a seal around the larynx. All of this ensures that the laryngeal mask airway is convenient and efficient to use.

US patent application 2004089307 discloses an artificial airway device comprising a laryngo-pharyngeal mask which includes an expandable masking ring of roughly elliptical shape. The expandable mask sealingly surrounds the laryngeal inlet when expanded, to obstruct communication between the laryngeal inlet and the esophagus. One or more airway tubes connected to the mask provide for fluid flow to a portion of the mask facing the laryngeal inlet when the mask sealingly surrounds the laryngeal inlet. A gastro-tube connected to the mask is bonded along most of its length to the airway tube and provides a fluid flow-path to the surface of the mask facing the esophagus when the mask sealingly surrounds the laryngeal inlet. The distal end of the gastro tube passes through the masking ring at its narrower distal region where, when installed in a patient, it abuts against the esophagus; and the distal portion of the gastro tube flattens when the mask is deflated to facilitate smooth passage behind the larynx during insertion through the mouth and throat of the patient.

US patent 4,681,094 discloses a laryngoscope comprising a disposable blade unit including two full length blades releasably interconnected along the length thereof to define a tubular passage. A reusable handle unit removably receives one end of the blade unit and retains the blades thereof. The blade unit includes an inflatable balloon expandable against the roof of the mouth to center an inserted laryngoscope and effect movement thereof against the tongue and epiglottis. A pair of suction channels are defined through the blade unit and communicated with the interior of the tubular passage at the leading end thereof. The handle unit includes means for communicating the balloon and suction passages with sources of positive and negative air pressure respectively. The handle unit also mounts illuminating means and a selectively usable adapter for accommodating a hand-manipulable air bag for emergency respiration directly through the laryngoscope.

PCT application WO2004006746 discloses a device for the lateral separation of the air passages of patients or for single-lung ventilation during thoracic surgery. The device comprises a laryngeal mask, upstream of the larynx of the patient, in which a first tube is integrated for lung ventilation. The aim of the invention is to develop a device which enables the lateral separation of the air passages of the patient, or single-lung ventilation, in a safe and effective manner, and which enables the disadvantages of the double lumen tube used until now and the bronchus blocker available until now to be avoided. According to this invention, in addition to the laryngeal mask in which the first tube is integrated, one such device comprises a second tube which can be introduced into the air passages via the first tube.

US patent application 2007/0163597 discloses an over-tube which guides a device having an insertion section to be insereted into a body of a patient.

GB patent application 2, 317,830 discloses a laryngeal mask assembly having a mask portion formed by a flexible bag enclosing a mount attached to the patient end of a tube.

US patent application 2007/0102001 discloses a laryngeal mask airway device including a mask portion and an airway tube.

These inventions are not guaranteed to prevent accidental insertion of the laryngeal mask into the esophagus or to prevent damage to the vocal cords. A laryngeal mask assembly that comprises active means for manipulating the tubing into the trachea still remains a long-standing but unmet need.

Furthermore, it is still a long-standing but unmet need to provide a laryngeal mask assembly without the need to detach the laryngeal mask. In other words, it would be advantageous if there were an integrated laryngeal mask assembly.

### SUMMARY OF THE INVENTION

The present invention relates to an integrated tubus and laryngeal mask device in accordance with amended appended independent claim 1 and appended dependent claims 2 to 16.

There is provided in accordance with a preferred embodiment of the present invention an integrated *(i)* tubus, and *(ii)* laryngeal mask device, comprising: (a)an outer laryngeal mask tube (OLT) having proximal portion and distal portion; comprising: (i)a balloon cuff shaped as a laryngeal mask, having at least one inflated configuration and at least one deflated configuration, located in the distal portion; the balloon in a fluid connection with an inflating means, located at the proximal end; and,(ii)an elongated tube in connection with the balloon cuff (TOLT);
(b)an inner tube (tubus) located within the OLT; the tubus is characterized by a main longitudinal axis; and,
(c)at least one lateral movement preventing element located circumferentially to the tubus and between the tubus and the OLT; adapted both *(i)* to prevent lateral movement of the tubus with respect to the OLT, such that contamination of the environment between the tubus and the OLT is prevented; and *(ii)* to allow longitudinal reciprocating movement of the tubus along the main longitudinal axis with respect to the OLT, in a manner that a seal is provided in the inner volume between the OLT and the tubus ,wherein, when the balloon cuff is in the deflated configuration, the thickness of the same is reduced such that the balloon cuff is not protruding from the TOLT.

There is further provided in accordance with a preferred embodiment of the present invention the integrated tubus and laryngeal mask device as defined above, additionally comprising a stopper adapted to fixedly secure the tubus to the operating position within the OLT.

There is further provided in accordance with a preferred embodiment of the present invention the integrated tubus and laryngeal mask device as defined above, additionally comprising a guide wire substantially accommodated along the length of the OLT; the guide wire is located in an inner canal of the laryngeal mask.
There is further provided in accordance with a preferred embodiment of the present invention the integrated tubus and laryngeal mask device as defined above, wherein the guide wire is adapted for affixing and stabilizing the laryngeal mask within patient's throat.

There is further provided in accordance with a preferred embodiment of the present invention the integrated tubus and laryngeal mask device as defined above, wherein the OLT can be easily peeled off by using the guide wire .

There is further provided in accordance with a preferred embodiment of the present invention the integrated tubus and laryngeal mask device as defined above, wherein the inflating means is a pilot balloon valve connected to an inflation lumen extending along the OLT and the tubus.

There is further provided in accordance with a preferred embodiment of the present invention the integrated tubus and laryngeal mask device as defined above, wherein the lateral movement preventing element is an O-ring.

There is further provided in accordance with a preferred embodiment of the present invention the integrated tubus and laryngeal mask device as defined above, wherein the sizes of the O-ring are specified by the outside diameter of the tubus and the cross sectional diameter between the tubus and the OLT.

There is further provided in accordance with a preferred embodiment of the present invention the integrated tubus and laryngeal mask device as defined above, wherein the O-ring is an elastomeric O-ring for sealing.

There is further provided in accordance with a preferred embodiment of the present invention the integrated tubus and laryngeal mask device as defined above, wherein the lateral movement preventing element is a balloon ring.

There is further provided in accordance with a preferred embodiment of the present invention the integrated tubus and laryngeal mask device as defined above, wherein the lateral movement preventing element is a gasket which has the structure and characteristic elements enabling the lateral movement preventing element to be placed between the tubus and the OLT.

There is further provided in accordance with a preferred embodiment of the present invention the integrated tubus and laryngeal mask device as defined above, wherein the sealing structure is selected from the group consisting of: a peripheral seal, a radial seal and any combination thereof.

There is further provided in accordance with a preferred embodiment of the present invention the integrated tubus and laryngeal mask device as defined above,
wherein the stopper prevents travel of the tubus lengthwise in the OLT.

There is further provided in accordance with a preferred embodiment of the present invention the introducer as defined above, additionally comprising a maneuvering mechanism adapted to reorient the distal end of the tubus.

There is further provided in accordance with a preferred embodiment of the present invention the integrated tubus and laryngeal mask device as defined above, wherein the OLT is further adapted for guiding a working tool to an operating position to view vocal cords and trachea of the patient.

There is further provided in accordance with a preferred embodiment of the present invention the integrated tubus and laryngeal mask device as defined above, further wherein the OLT guides a working tool in a manner which allows ventilation through the tubus to continue without interruption.

There is further provided in accordance with a preferred embodiment of the present invention the integrated tubus and laryngeal mask device as defined above, additionally comprising a second inflatable balloon located at the distal end of the tubus.

There is further provided in accordance with a preferred embodiment of the present invention the integrated tubus and laryngeal mask device as defined above, additionally comprising a second valve for controlling inflation and deflation of the second balloon. There is further provided in accordance with a preferred embodiment of the present invention the integrated tubus and laryngeal mask device as defined above,wherein the second balloon is made of a flexible material.

There is further provided in accordance with a preferred embodiment of the present invention the integrated tubus and laryngeal mask device as defined above, wherein the balloon cuff is inflated to its first state when the laryngeal mask is inserted, such that it blocks entry into the esophagus of the TOLT.

There is further provided in accordance with a preferred embodiment of the present invention the integrated tubus and laryngeal mask device as defined above, wherein the first state is a state of the tubus which prevents entry of the esophagus into the internal cavity within the OLT.

There is further provided in accordance with a preferred embodiment of the present invention the integrated tubus and laryngeal mask device as defined above, wherein the second inflatable balloon is disposed about one side of, and in physical contact with, the tubus.

There is further provided in accordance with a preferred embodiment of the present invention the integrated tubus and laryngeal mask device as defined above, wherein the second balloon is in physical contact with one side of the tubus, whereby upon inflation of the second balloon while the integrated tubus and laryngeal mask device is in place, at least part of the tubus is positioned in a predetermined direction.

There is further provided in accordance with a preferred embodiment of the present invention the integrated tubus and laryngeal mask device as defined above, wherein the predetermined direction is in the direction of the trachea of the patient.

There is further provided in accordance with a preferred embodiment of the present invention the integrated tubus and laryngeal mask device as defined above, wherein the OLT has flexible walls integral with the balloon cuff of the OLT and the two are in contact when the balloon cuff is deflated.

There is further provided in accordance with a preferred embodiment of the present invention the integrated tubus and laryngeal mask device as defined above, wherein the balloon cuff is deflated to its second state once the tubus is inserted to the correct position, such that it provides no substantial obstruction within the OLT.

There is further provided in accordance with a preferred embodiment of the present invention the integrated tubus and laryngeal mask device as defined above, wherein the second state is a state in which the internal cavity within the OLT is substantially open.

There is further provided in accordance with a preferred embodiment of the present invention the integrated tubus and laryngeal mask device as defined above, wherein the balloon cuff is an expansible member made of an elastic material.

There is further provided in accordance with a preferred embodiment of the present invention the integrated tubus and laryngeal mask device as defined above, wherein the balloon cuff is attached to the OLT and communicates with an inflation lumen extending along the OLT and the tubus.

There is further provided in accordance with a preferred embodiment of the present invention the integrated tubus and laryngeal mask device as defined above, wherein the OLT is fixedly secured to the operating position of the tubus.

There is further provided in accordance with a preferred embodiment of the present invention the integrated tubus and laryngeal mask device as defined above, wherein the second inflatable balloon is disposed externally to the flexible airway.

There is further provided in accordance with a preferred embodiment of the present invention the integrated tubus and laryngeal mask device as defined above, wherein the second inflatable balloon is disposed within the flexible airway.

There is further provided in accordance with a preferred embodiment of the present invention the integrated tubus and laryngeal mask device as defined above, wherein the second inflatable balloon is integrated within the flexible airway.

There is provided a method, not forming part of the invention, for intubating a patient using an integrated tubus and laryngeal mask device, the method comprising a step of: (a)obtaining an integrated *(i)* tubus and, *(ii)* laryngeal mask device, comprising: (i)an outer laryngeal mask tube (OLT) having proximal end and distal portion; comprising: (1)a laryngeal mask with integral balloon cuff, having at least one inflated configuration and at least one deflated configuration, located in the distal portion and in fluid connection with an inflating means located at the proximal end of the OLT; and,(2) a longitudinal tube in communication with the balloon cuff;
(ii) an inner tube (tubus) located within the OLT; the tubus characterized by a main longitudinal axis; and,
(iii) at least one lateral movement preventing element located circumferentially to the tubus and between the tubus and the OLT; adapted to both *(i)* prevent lateral movement of the tubus with respect to the OLT such that a contact-free environment between the tubus and the OLT is provided; and *(ii)* allow longitudinal reciprocating movement of the tubus along the main longitudinal axis with respect to the OLT; sealing the inner volume between the tube of the OLT and the tubus such that contamination of the volume is prevented;

(b) inserting the laryngeal mask in its deflated configuration into the mouth of a patient;(c) positioning the laryngeal mask adjacent to the larynx, (d) inflating the inserted balloon cuff in patient's larynx, (e) fixedly securing the tubus to the distal portion of the OLT and (f) deflating the balloon cuff, wherein, when the balloon cuff is deflated, the thickness of the same is reduced such that the balloon cuff is substantially in contact with the tube of the OLT.

There is further provided a method, not forming part of the invention, as defined above, additionally comprising a step of initiating ventilation of the patient via the proximal end.

There is further provided a method, not forming part of the invention, as defined above, additionally comprising a step of moving a working tool through the OLT.

There is further provided a method, not forming part of the invention, as defined above, additionally comprising a step of moving the working tool beyond the mask and into the trachea of the patient.

There is further provided a method, not forming part of the invention, as defined above, wherein the integrated tubus and laryngeal mask device further comprises an airway-directing mechanism disposed about one side of the tubus, the method further comprising a step of at least partially maneuvering the airway-directing mechanism whereby the tubus is directed toward the trachea of the patient.

There is further provided a method, not forming part of the invention, as defined above, additionally comprising a step of fixedly securing the OLT to the operating position of the tubus.

There is further provided a method, not forming part of the invention, as defined above, wherein the OLT is further adapted for guiding the working tool to an operating position to view vocal cords and trachea of the patient.

There is further provided a method, not forming part of the invention, as defined above, additionally comprising a step of providing a second balloon disposed about, and in physical contact with, one side of the tubus.

There is further provided a method, not forming part of the invention, as defined above, additionally comprising a step of, upon inflation of the inflatable balloon while the tubus is in place, directing at least part of the integrated tubus and laryngeal mask device in the direction of the trachea of the patient.

There is further provided a method, not forming part of the invention, as defined above, additionally comprising a step of disposing the second inflatable balloon externally to the tubus.

There is further provided a method, not forming part of the invention, as defined above, additionally comprising a step of disposing the second inflatable balloon within the flexible airway.

There is further provided a method, not forming part of the invention, as defined above, additionally comprising step of maneuvering and reorienting the distal end of the tubus.

There is further provided a method, not forming part of the invention, as defined above, additionally comprising a stopper adapted to fixedly secure the tubus in the operating position of the tubus. There is further provided a method, not forming part of the invention, as defined above, additionally comprising a guide wire substantially accommodated along the length of the OLT; the guide wire is located in an inner canal of the laryngeal mask.
There is further provided a method, not forming part of the invention, as defined above, wherein the guide wire is adapted for affixing and stabilizing the laryngeal mask within patient's throat.
There is further provided a method, not forming part of the invention, as defined above, wherein the OLT can be easily peeled off by using the guide wire.

There is further provided a method, not forming part of the invention, as defined above, wherein the valve is a pilot balloon connected to an inflation lumen extending along the OLT and the tubus.

There is further provided a method, not forming part of the invention, as defined above, wherein the lateral movement preventing element is an O-ring.

There is further provided a method, not forming part of the invention, as defined above, wherein the O-rings has sizes specified by the outside diameter of the tubus and the cross sectional diameter between the tubus and the OLT.

There is further provided a method, not forming part of the invention, as defined above, wherein the O-ring is an elastomeric O-ring for sealing.

There is further provided a method, not forming part of the invention, as defined above, wherein the lateral movement preventing element is ring-type balloon.

There is further provided a method, not forming part of the invention, as defined above, wherein the lateral movement preventing element is any type of a gasket which has the structure and characteristics to be placed between the tubus and the OLT.

There is further provided a method, not forming part of the invention, as defined above, wherein the sealing structure is selected from the group consisting of: a peripheral seal, a radial seal, and any combination thereof.

There is further provided a method, not forming part of the invention, as defined above, wherein the stopper is adapted to prevent travel of the tubus lengthwise in the OLT.

There is further provided a method, not forming part of the invention, as defined above, wherein the stopper has size, shape and mechanism to reinforce the tubus.

There is further provided a method, not forming part of the invention, as defined above, additionally comprising a second valve for controlling inflation and deflation of the second balloon.

There is further provided a method, not forming part of the invention, as defined above, wherein the balloon cuff is made of a flexible material.

There is further provided a method, not forming part of the invention, as defined above, wherein, when inserting the tubus, the inflation of the balloon cuff to its first state blocks entry of the tubus into the esophagus.

There is further provided a method, not forming part of the invention, as defined above, wherein the first state is a state of the integrated tubus and laryngeal mask device which prevents entry of the esophagus into the internal cavity within the mask.

There is further provided a method, not forming part of the invention, as defined above, wherein the second balloon is in physical contact with one side of the tubus, whereby, upon inflation of the second balloon while the OLT is in place, at least part of the tubus is directed in a predetermined direction.

There is further provided a method, not forming part of the invention, as defined above, wherein the OLT tube has flexible walls integral with the cuff balloon of the OLT; the OLT walls and the cuff balloon walls being substantially in contact when the cuff balloon is deflated.

There is further provided a method, not forming part of the invention, as defined above, wherein the balloon is deflated to its second state once inserted to and secured in the correct position, such that it provides no substantial obstruction within the mask.

There is further provided a method, not forming part of the invention, as defined above, wherein the second state is a state in which the internal cavity within the mask is substantially open.

There is further provided a method, not forming part of the invention, as defined above, wherein the second balloon and balloon cuff are expansible members made of an elastic material.

There is provided a method, not forming part of the invention, of producing an integrated tubus and laryngeal mask device, comprising steps of: (a)providing an outer laryngeal mask tube (OLT) having proximal end and distal portion having: *(i)* a balloon cuff shaped as a laryngeal mask, having at least one inflated configuration and at least one deflated configuration, located in the distal portion; the balloon in a fluid connection with an inflating means, located at the proximal end, and an elongated tube in connection with the balloon cuff (TOLT); *(ii)* an inner tube (tubus); the tubus characterized by a main longitudinal axis; *(iii)* at least one lateral movement preventing element, (b)locating the tubus within the OLT (c)positioning the lateral movement preventing element circumferentially to the tubus and between the tubus and the OLT, wherein, the balloon cuff in its deflated configuration is not protruding from the TOLT.

There is further provided a method, not forming part of the invention, as defined above, wherein the lateral movement preventing element is adapted both *(i)* to prevent lateral movement of the tubus with respect to the OLT, such that contamination of the environment between the tubus and the OLT is prevented; and *(ii)* to allow longitudinal reciprocating movement of the tubus along the main longitudinal axis with respect to the OLT, in a manner that a seal is provided for the inner volume between the OLT and the tubus.

There is further provided a method, not forming part of the invention, as defined above, additionally comprising the step of positioning a stopper at the proximal end of the OLT.

There is further provided a method, not forming part of the invention, as defined above, additionally comprising a guide substantially accommodated along the length of the OLT; the guide wire is located in an inner canal of the laryngeal mask.
There is further provided a method, not forming part of the invention, as defined above, wherein the guide wire is adapted for affixing and stabilizing the laryngeal mask within patient's throat.
There is further provided a method, not forming part of the invention, as defined above, wherein the OLT can be easily peeled off by using the guide wire .

There is further provided a method, not forming part of the invention, as defined above, wherein the tubus additionally comprises a second inflatable balloon.

There is further provided a method, not forming part of the invention, as defined above, additionally comprising the step of sealing the inner volume between the OLT and the tubus with the lateral movement preventing element.

There is further provided a method, not forming part of the invention, as defined above, wherein the seal is a peripheral seal.

There is further provided a method, not forming part of the invention, as defined above, wherein the seal is a radial seal.

There is further provided a method, not forming part of the invention, as defined above, wherein the inflating means is a pilot balloon valve connected to an inflation lumen extending along the OLT and the tubus.

There is further provided a method, not forming part of the invention, as defined above, wherein the second balloon is in fluid connection with a second inflating means located at the proximal end of the tubus.

There is further provided a method, not forming part of the invention, as defined above, wherein the second inflating means is a pilot balloon valve connected to an inflation lumen extending along the OLT and the tubus.

There is further provided a method, not forming part of the invention, as defined above, further wherein the inflating means located at the proximal end is in fluid connection with the lateral movement preventing element.

### BRIEF DESCRIPTION OF THE DRAWINGS

In order to understand the invention and to see how it may be implemented in practice, a few preferred embodiments will now be described, by way of non-limiting example only, with reference to be accompanying drawings, in which:
**FIG. 1** is a cross-sectional view of a laryngeal mask assembly according to one embodiment of the invention;
**FIG. 2** is a cross-sectional view of a laryngeal mask assembly in its operating position according to a second embodiment of the invention;
**FIG. 3** is a cross-sectional view, of a laryngeal mask assembly in its operating position according to the embodiment of the invention illustrated in FIG. **2**;
**FIG 4** illustrates a flow chart of a method for intubating a patient using a laryngeal mask assembly; and
**FIG 5** illustrates a flow chart of a method for producing a laryngeal mask assembly.

### DETAILED DESCRIPTION OF THE PREFERRED EMBODIMENTS

In the following description, various aspects of the invention will be described. For the purposes of explanation, specific details are set forth in order to provide a thorough understanding of the invention. It will be apparent to one skilled in the art that there are other embodiments of the invention that differ in details without affecting the essential nature thereof. Therefore the invention is not limited by that which is illustrated in the figures and described in the specification, but only as indicated in the accompanying claims, with the proper scope determined only by the broadest interpretation of the claims.

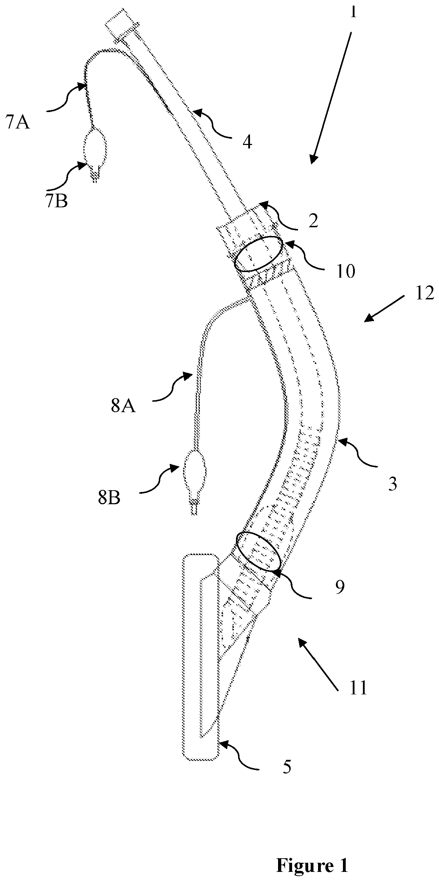

Reference is now made to **FIG. 1**, which shows a cross-sectional view of an artificial airway assembly before insertion according to one embodiment **1** of the present invention.

In preferred embodiments, the integrated tubus and laryngeal mask device **1** comprises an outer laryngeal mask tube (OLT) **4** having proximal portion **12** and distal portion **11**. The OLT comprises a balloon cuff **5** in a communication with a longitudinal tube **3**. The balloon cuff **5** is shaped as a laryngeal mask, having at least one inflated configuration and at least one deflated configuration, and is in fluid connection with an inflating means. The balloon cuff is located at the distal end **11** of the OLT. The balloon cuff is an expansible member, made of an elastic material, which has an approximately toroidal shape.

The integrated tubus and laryngeal mask device **1** further comprises a flexible airway tube (i.e., a tubus) **4** as an inner tube (tubus) located within the OLT 3. The tubus is characterized by a main longitudinal axis. The balloon cuff is connected to an inflation lumen **8A** extending along the OLT and the tubus with an inflatable valve **8B**, such as a pilot balloon, located at the inflation lumen edge. The valve assembly **8B** is in fluid connection with the OLT and allows air to pass in and out of the OLT balloon cuff, allowing for easier insertion, as the OLT balloon cuff need not be inflated until it is already substantially in place.

The OLT is adapted to conform to the space immediately adjacent to the larynx of the patient. In preferred embodiments, the OLT is constructed of a resilient flexible biocompatible material in the form of a peripheral ring capable of defining a hollow space, capable of being inflated and deflated to accommodate the space adjacent to the larynx. When installed, the OLT adopts a configuration substantially conforming to the shape of the laryngeal inlet.

The integrated tubus and laryngeal mask device **1** further comprises at least one lateral movement preventing element **9-10** located circumferentially to the tubus and between the inner lumen of the tubus and the OLT. The lateral movement preventing element is adapted to prevent lateral movement of the tubus with respect to the OLT such that a contact-free environment between the tubus and the OLT is provided. The lateral movement preventing element is further adapted to allow longitudinal reciprocating movement of the tubus along the main longitudinal axis with respect to the OLT. The main goal of the lateral movement preventing element is to create a peripheral sealing or a radial sealing of the inner volume between the tube of the OLT and the tubus, preventing any contamination of the inner tube or infection of the throat or esophagus. The lateral movement preventing element **9-10** is selected from the group consisting of: an O ring, an inflated balloon ring or any gasket which has the structure and character to create a peripheral seal or a radial seal in between the tubus and the OLT. The O-ring acts as a mechanical gasket. It has the shape of a torus, and is designed as a loop of elastomer with a disc-shaped cross-section. The lateral movement preventing element can be positioned in the distal portion or/and in the proximal portion of the device. The lateral movement preventing element can be activated by an inflation valve assembly **7** if inflation or deflation of the element is required.

The integrated tubus and laryngeal mask device **1** further comprises a stopper **2** as an engagement part located in the proximal portion. The stopper fixedly secures the tubus to an operating position inside the OLT. The stopper prevents the tubus from traveling lengthwise in the OLT tube when ventilation is performed.

The integrated tubus and laryngeal mask device **1** further comprises a guide wire substantially accommodated along the length of the OLT. The guide wire is adapted for immutability and easy intubation of the laryngeal mask by providing a stiffness characters to the OLT. Further more, the guide wire enables the laryngeal mask to be flexible and rigid simultaneously such that it will not collapse or fold when inserted to patient's delivery system. The guide wire is positioned within an inner canal of the laryngeal mask.

The guide wire is further adapted for affixing and stabilizing the laryngeal mask within patient's throat. Further more, the OLT can be easily removed using the guide wire such that the OLT is peeled off from patient's throat whilst the tubus remains within the patient's throat.

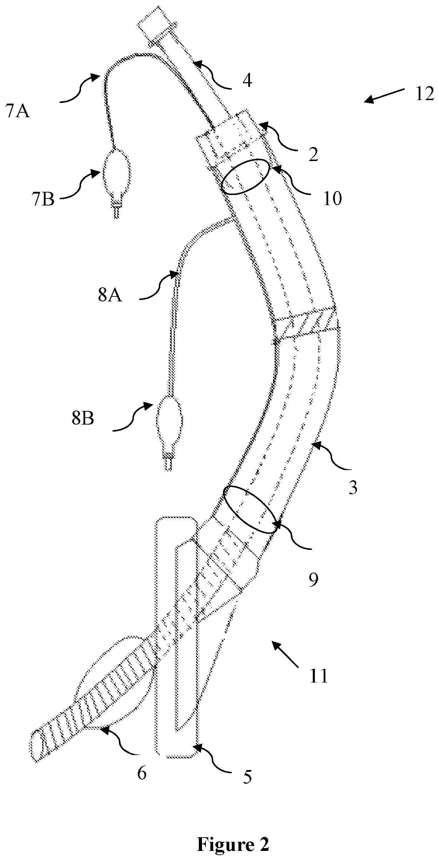

Reference is now made to **FIG. 2****,** which shows a cross-sectional view of the integrated tubus and laryngeal mask device of a second embodiment of the invention. In this embodiment, the tubus is inserted into patient's trachea until it is substantially in place order to intubate a patient. The tubus **4** is inserted into the mouth of the patient. The OLT **5** is positioned adjacent to the larynx, thus sealing at least partially the integrated tubus and laryngeal mask device for communication exclusively through the laryngeal inlet.

In another embodiment of the invention, air is injected into the OLT balloon cuff **5** via the valve assembly **8A-8B**. The distal end of the fully inflatable cuff formation prevents the more rigid distal end of the OLT from catching the inside of the throat and subjecting the patient to undesirable forces.

In another embodiment of the invention, a valve assembly **7A-7B** is presented. The valve assembly **7A-7B** comprises an inflation lumen **7A** and an operation valve **7B** located within the edge of the inflation lumen. The valve assembly is in physical connection with the tubus. The valve **7A-7B** controls the inflating and deflating activation respectively of a second balloon attached to the distal end of the tubus when the integrated tubus and laryngeal mask device is substantially in place. Air can be also injected into the second balloon **6** by use of a syringe, but any means known in the art may be used instead.

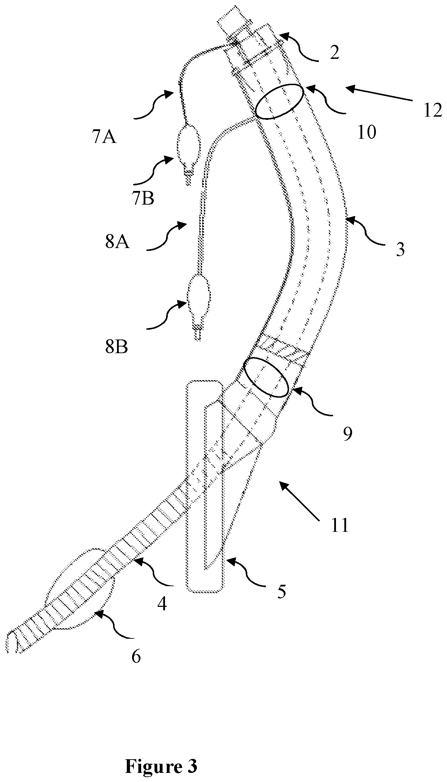

Reference is now made to **FIG. 3****,** which shows a cross-sectional view of the integrated tubus and laryngeal mask device of a third embodiment of the invention. In this embodiment, the tubus is in its operating position as it is used to intubate a patient. The OLT balloon cuff **5** is positioned adjacent to the larynx, thus sealing at least partially the integrated tubus and laryngeal mask device for communication exclusively through the laryngeal inlet as it is positioned inside the trachea in order to start the ventilation process on a patient. When the laryngeal mask is in its insertion position, the balloon cuff **5** is inflated to its first state so that it blocks entry into the esophagus of the TOLT. Once inserted to the correct position, the balloon cuff **5** is deflated to its second state so that it provides no substantial obstruction within the mask 5.

In **FIG. 3**, the tubus is fixedly secured inside the patient's trachea thus allowing deflation of the balloon cuff using the valve assembly **8A-8B**. When air is withdrawn and released from the inflation lumen **8A**, the resilience of the balloon **5** causes it to retract. Its thickness is reduced so that the balloon cuff lies flat against the surface of the OLT and adjacent to the OLT tube's walls, resulting an integration of the cuff with the tube of the OLT. In a preferred embodiment, the OLT tube is integrated with a deflated cuff balloon (masking ring) which provides a seal around the laryngeal inlet with an adjacent independent airway supply to the patient's lungs.

In another embodiment of the invention, **FIG 3** further presents a second balloon **6** disposed about one side, which is inflated by supplying air or other fluid along the inflation lumen **7A** in the usual way, so that the balloon expands to substantially fill the cavity of the trachea. The second balloon **6** is in physical contact with the tubus' distal end. The second balloon **6** may be of any appropriate biocompatible rubber-like material. In preferred embodiments of the invention, it is disposed on the outer circumference of the tubus. In other embodiments, it is disposed within tubus.

After insertion of the integrated tubus and laryngeal mask device, an additional working tool may be inserted through the proximal portion of the OLT and into the trachea in a position from which a viewing device (e.g. a fiber optic device) can be used to observe the vocal cords.

In another embodiment, the artificial airway assembly defined above is provided with a pneumatic airway-directing mechanism which comprises at least one inflatable balloon useful to direct at least part of the integrated tubus and laryngeal mask device to a predetermined location. Additionally, or alternatively, the integrated tubus and laryngeal mask device defined above, is provided with a mechanical airway-directing mechanism which comprises at least one maneuverable effecter useful to direct at least part of the flexible airway to a predetermined location. The integrated tubus and laryngeal mask device which comprises an integrated mechanism, namely a pneumatic and mechanical mechanism, is possible.

It is another object of the present invention to provide an integrated *(i)* tubus, and *(ii)* laryngeal mask device, comprising: *(a)* an outer laryngeal mask tube (OLT) having proximal portion and distal portion; comprising: *(i)* a balloon cuff shaped as a laryngeal mask, having at least one inflated configuration and at least one deflated configuration, located in the distal portion. The balloon is in a fluid connection with an inflating means, located at the proximal end; and, *(ii)* an elongated tube in connection with the balloon cuff (TOLT); *(b)* an inner tube (tubus) located within the OLT; the tubus is characterized by a main longitudinal axis; and, *(c)* at least one lateral movement preventing element located circumferentially to the tubus and between the tubus and the OLT; adapted both *(i)* to prevent lateral movement of the tubus with respect to the OLT, such that contamination of the environment between the tubus and the OLT is prevented; and *(ii)* to allow longitudinal reciprocating movement of the tubus along the main longitudinal axis with respect to the OLT, in a manner that a seal is provided in the inner volume between the OLT and the tubus; wherein, when the balloon cuff is in the deflated configuration, the thickness of the same is reduced such that the balloon cuff is not protruding from the TOLT.

It is another object of the present invention to provide an integrated tubus and laryngeal mask device, additionally comprising a stopper adapted to fixedly secure the tubus to the operating position within the OLT.

It is another object of the present invention to provide an integrated tubus and laryngeal mask device, additionally comprising a guide wire substantially accommodated along the length of the OLT; the guide wire is located in an inner canal of the laryngeal mask.

It is another object of the present invention to provide an integrated tubus and laryngeal mask device as defined above, wherein the inflating means is a pilot balloon valve connected to an inflation lumen extending along the OLT and the tubus.

It is another object of the present invention to provide an integrated tubus and laryngeal mask device as defined above, wherein the lateral movement preventing element is an O-ring.

It is another object of the present invention to provide an integrated tubus and laryngeal mask device as defined above, wherein the sizes of the O-rings are specified by the outside diameter of the tubus and the cross sectional diameter between the tubus and the OLT.

It is another object of the present invention to provide an integrated tubus and laryngeal mask device as defined above, wherein the O-ring is an elastomeric O-ring for sealing.

It is another object of the present invention to provide an integrated tubus and laryngeal mask device as defined above, wherein the lateral movement preventing element is a balloon ring.

It is another object of the present invention to provide an integrated tubus and laryngeal mask device as defined above, wherein the lateral movement preventing element is any type of gasket which has the structure and characteristic elements enabling the lateral movement preventing element to be placed between the tubus and the OLT.

It is another object of the present invention to provide an integrated tubus and laryngeal mask device as defined above, wherein the sealing structure is selected from the group consisting of a peripheral seal, a radial seal, and any combination thereof.

It is another object of the present invention to provide an integrated tubus and laryngeal mask device as defined above, wherein the stopper prevents travel of the tubus lengthwise in the OLT.

It is another object of the present invention to provide an integrated tubus and laryngeal mask device as defined above, additionally comprising a maneuvering mechanism adapted to reorient the distal end of the tubus.

It is another object of the present invention to provide an integrated tubus and laryngeal mask device as defined above, wherein the OLT is further adapted for guiding a working tool to an operating position to view vocal cords and trachea of the patient.

It is another object of the present invention to provide an integrated tubus and laryngeal mask device as defined above, further wherein the OLT guides a working tool in a manner which allows ventilation through the tubus to continue without interruption.

It is another object of the present invention to provide an integrated tubus and laryngeal mask device as defined above, additionally comprising a second inflatable balloon located at the distal end of the tubus.

It is another object of the present invention to provide an integrated tubus and laryngeal mask device as defined above, additionally comprising a second valve for controlling inflation and deflation of the second balloon.

It is another object of the present invention to provide an integrated tubus and laryngeal mask device as defined above, wherein the second balloon is made of a flexible material.

It is another object of the present invention to provide an integrated tubus and laryngeal mask device as defined above, wherein the balloon cuff is inflated to its first state when the laryngeal mask is inserted, such that it blocks entry into the esophagus of the TOLT.

It is another object of the present invention to provide an integrated tubus and laryngeal mask device as defined above, wherein the first state is a state of the tubus which prevents entry of the esophagus into the internal cavity within the OLT.

It is another object of the present invention to provide an integrated tubus and laryngeal mask device as defined above, wherein the inflatable balloon is disposed about one side of, and in physical contact with, the tubus.

It is another object of the present invention to provide an integrated tubus and laryngeal mask device as defined above, wherein the second balloon is in physical contact with one side of the tubus, whereby upon inflation of the second balloon while the integrated tubus and laryngeal mask device is in place, at least part of the tubus is positioned in a predetermined direction.

It is another object of the present invention to provide an integrated tubus and laryngeal mask device as defined above, wherein the predetermined direction is in the direction of the trachea of the patient.

It is another object of the present invention to provide an integrated tubus and laryngeal mask device as defined above, wherein the OLT has flexible walls integral with the balloon cuff of the OLT and the two are substantially in contact when the balloon cuff is deflated.

It is another object of the present invention to provide an integrated tubus and laryngeal mask device as defined above, wherein the balloon cuff is deflated to its second state once the tubus is inserted to the correct position, such that it provides no substantial obstruction within the OLT.

It is another object of the present invention to provide an integrated tubus and laryngeal mask device as defined above, wherein the second state is a state in which the internal cavity within the OLT is substantially open.

It is another object of the present invention to provide an integrated tubus and laryngeal mask device as defined above, wherein the balloon cuff is an expansible member made of an elastic material.

It is another object of the present invention to provide an integrated tubus and laryngeal mask device as defined above, wherein the balloon cuff is attached to the OLT and communicates with an inflation lumen extending along the OLT and the tubus.

It is another object of the present invention to provide an integrated tubus and laryngeal mask device as defined above, wherein the OLT is fixedly secured to the operating position of the tubus.

It is another object of the present invention to provide an integrated tubus and laryngeal mask device as defined above, wherein the second inflatable balloon is disposed externally to the flexible airway.

It is another object of the present invention to provide an integrated tubus and laryngeal mask device as defined above, wherein the second inflatable balloon is disposed within the flexible airway.

It is another object of the present invention to provide an integrated tubus and laryngeal mask device as defined above, wherein the second inflatable balloon is integrated within the flexible airway.

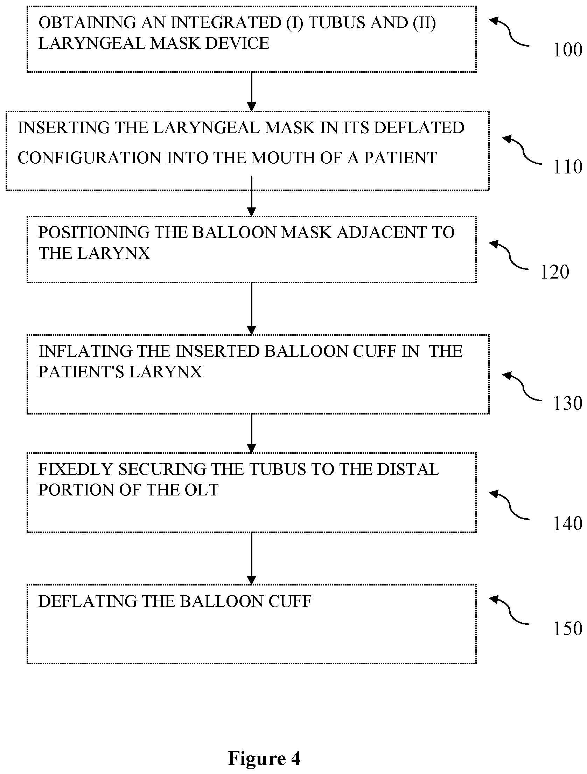

**Fig 4** illustrates a flow chart of a method for intubating a patient using a laryngeal mask assembly. It is a method for intubating a patient using an integrated tubus and laryngeal mask device, the method comprising a step of: (a) obtaining an integrated (I) tubus and (II) laryngeal mask device **100**, comprising: (i) an outer laryngeal mask tube (OLT) having proximal end and distal portion, comprising: (a) a laryngeal mask with integral balloon cuff, having at least one inflated configuration and at least one deflated configuration, located in the distal portion and in fluid connection with an inflating means located at the proximal end of the OLT; and, (b) a longitudinal tube in communication with the balloon cuff;
(ii) an inner tube (tubus) located within the OLT, the tubus characterized by a main longitudinal axis, and (iii) at least one lateral movement preventing element located circumferentially to the tubus and in between the tubus and the OLT; adapted to both (i) prevent lateral movement of the tubus with respect to the OLT such that a contact-free environment between the tubus and the OLT is provided, and (*ii*) allow longitudinal reciprocating movement of the tubus along the main longitudinal axis with respect to the OLT; sealing of the inner volume between the tube of the PLT and the tubus such that contamination of the volume is prevented;

(b) inserting the laryngeal mask in its deflated configuration into the mouth of a patient **110**, (c) positioning the balloon mask adjacent to the larynx **120**, (d) inflating the inserted balloon cuff in patient's larynx **130**, (e) fixedly securing the tubus to the distal portion of the OLT **140**; and (f) deflating the balloon cuff **150**;
   wherein, when balloon cuff is deflated, the thickness of the same is reduced such that the balloon cuff is substantially in contact with the tube of the OLT.

It is provided a method for intubating a patient using an integrated tubus and laryngeal mask device additionally comprising a step of initiating ventilation of the patient via the proximal end.

It is provided a method for intubating a patient using an integrated tubus and laryngeal mask device, additionally comprising a step of moving a working tool through the OLT

It is provided a method as defined above, additionally comprising a step of moving the working tool beyond the mask and into the trachea of the patient.

It is provided a method as defined above, wherein the integrated tubus and laryngeal mask device further comprises an airway-directing mechanism disposed about one side of the tubus, the method further comprising a step of at least partially maneuvering the airway-directing mechanism whereby the tubus is directed toward the trachea of the patient.

It is provided a method as defined above, additionally comprising a step of fixedly securing the OLT to the operating position of the tubus.

It is provided a method as defined above, wherein the OLT is further adapted for guiding the working tool to an operating position to view vocal cords and trachea of the patient.

It is provided a method as defined above, additionally comprising a step of providing a second balloon disposed about, and in physical contact with, one side of the tubus.

It is provided a method as defined above, additionally comprising a step of, upon inflation of the inflatable balloon while the tubus is in place, directing at least part of the integrated tubus and laryngeal mask device in the direction of the trachea of the patient.

It is provided a method as defined above, additionally comprising a step of disposing the second inflatable balloon externally to the tubus.

It is provided a method as defined above, additionally comprising a step of disposing the second inflatable balloon within the flexible airway.

It is provided a method as defined above, additionally comprising step of maneuvering and reorienting the distal end of the tubus.

It is provided a method as defined above, additionally comprising a stopper adapted to fixedly secure the tubus in its operating position.

It is provided a method as defined above, additionally comprising a guide wire substantially accommodated along the length of the OLT; the guide wire is located in an inner canal of the laryngeal mask.

It is provided a method as defined above, wherein the valve is a pilot balloon connected to an inflation lumen extending along the OLT and the tubus.

It is provided a method as defined above, wherein the lateral movement preventing element is an O-ring.

It is provided a method as defined above, wherein the O-ring has sizes specified by the outside diameter of the tubus and the cross sectional diameter between the tubus and the OLT.

It is provided a method as defined above, wherein the O-ring is an elastomeric O-ring for sealing.

It is provided a method as defined above, wherein the lateral movement preventing element is ring-type balloon.

It is provided a method as defined above, wherein the lateral movement preventing element is any type of a gasket which has the structure and characteristics to be placed between the tubus and the OLT.

It is provided a method as defined above, wherein the sealing structure is selected from the group consisting of: a peripheral seal, a radial seal, and any combination thereof.

It is provided a method as defined above, wherein the stopper adapted to prevent travel of the tubus lengthwise in the OLT.

It is provided a method as defined above, wherein the stopper has the size, shape and mechanism to reinforce the tubus.

It is provided a method as defined above, additionally comprising a second valve for controlling inflation and deflation of the second balloon.

It is provided a method as defined above, wherein the balloon cuff is made of a flexible material.

It is provided a method as defined above, wherein, when inserting the tubus, inflation of the balloon cuff to its first state blocks entry of the tubus into the esophagus.

It is provided a method as defined above, wherein the first state is a state of the integrated tubus and laryngeal mask device which prevents entry of the esophagus into the internal cavity within the mask.

It is provided a method as defined above, wherein the second balloon is in physical contact with one side of the tubus, whereby, upon inflation of the second balloon while the OLT is in place, at least part of the tubus is directed in a predetermined direction.

It is provided a method as defined above, wherein the OLT tube has flexible walls integral with the cuff balloon of the OLT and substantially in contact with them when the cuff balloon is deflated.

It is provided a method as defined above, wherein the balloon is deflated to its second state once inserted to and secured in the correct position, such that it provides no substantial obstruction within the mask.

It is provided a method as defined above, wherein the second state is a state in which the internal cavity within the mask is substantially open.

It is provided a method as defined above, wherein the second balloon and the balloon cuff are expansible members made of an elastic material.

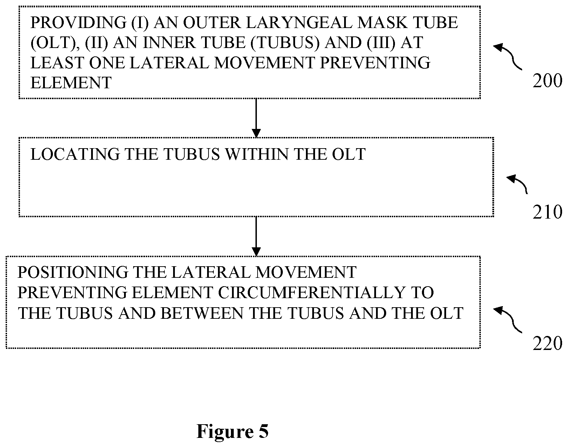

**Fig 5** illustrates a flow chart of a method for producing a laryngeal mask assembly. It is provided a method of producing an integrated tubus and laryngeal mask device as defined above, comprising steps of: *(a)* providing an outer laryngeal mask tube (OLT) having proximal end and distal portion **200** having: *(i)* a balloon cuff shaped as a laryngeal mask, having at least one inflated configuration and at least one deflated configuration, located at the distal portion; the balloon in fluid connection with an inflating means, located at the proximal end, and an elongated tube in connection with the balloon cuff (TOLT); *(ii)* an inner tube (tubus); the tubus characterized by a main longitudinal axis; *(iii)* at least one lateral movement preventing element; *(b)* locating the tubus within the OLT **210**, *(c)* positioning the lateral movement preventing element circumferentially to the tubus and in between the tubus and the OLT **220**; wherein the balloon cuff in its deflated configuration is not protruding from the TOLT.

It is provided a method as defined above, wherein the lateral movement preventing element is adapted both *(i)* to prevent lateral movement of the tubus with respect to the OLT, such that contamination of the environment between the tubus and the OLT is prevented; and *(ii)* to allow longitudinal reciprocating movement of the tubus along the main longitudinal axis with respect to the OLT, in a manner that a seal is provided for the inner volume between the OLT and the tubus.

It is provided a method as defined above, additionally comprising the step of positioning a stopper at the proximal end of the OLT.

It is provided a method as defined above, wherein the tubus additionally comprises a second inflatable balloon.

It is provided a method as defined above, additionally comprising the step of sealing the inner volume between the OLT and the tubus with the lateral movement preventing element.

It is provided a method as defined above, wherein the seal is a peripheral seal.

It is provided a method as defined above, wherein the seal is a radial seal.

It is provided a method as defined above, wherein the inflating means is a pilot balloon valve connected to an inflation lumen extending along the OLT and the tubus.

It is provided a method as defined above, wherein the second balloon is in fluid connection with a second inflating means located at the proximal end of the tubus.

It is provided a method as defined above, wherein the second inflating means is a pilot balloon valve connected to an inflation lumen extending along the OLT and the tubus.

It is provided a method as defined above, further wherein the inflating means located at the proximal end is in fluid connection with the lateral movement preventing element.

## Claims

1. An integrated (i) tubus, and (ii) laryngeal mask device (1), comprising:
a. an outer laryngeal mask tube (OLT) (3) having proximal portion (12) and distal portion (11); comprising:
i. a balloon cuff (5) shaped as a laryngeal mask, having at least one inflated configuration and at least one deflated configuration, located in said distal portion (11); said balloon in a fluid connection with an inflating means (8A, 8B), located at said proximal end; and,
ii. an elongated tube in connection with said balloon cuff (TOLT);
b. an inner tube (tubus) (4) located within said OLT (3), and allowing ventilation through the tubus; said tubus (4) is **characterized**
**by** a main longitudinal axis; and,
c. at least one lateral movement preventing element (9, 10) located circumferentially to said tubus (4) and between said tubus (4) and said OLT (3); adapted both (i) to prevent lateral movement of said tubus with respect to said OLT, such that contamination of the environment between said tubus and said OLT is prevented; and (ii) to allow longitudinal reciprocating movement of said tubus along said main longitudinal axis with respect to said OLT, in a manner that a seal is provided in the inner volume between the OLT and the tubus
wherein, when said balloon cuff (5) is in said deflated configuration, the thickness of the same is reduced such that said balloon cuff (5) is not protruding from said TOLT.

2. The integrated tubus and laryngeal mask device (1) according to claim 1, additionally comprising one element selected from a group consisting of
a. a stopper (2) adapted to fixedly secure said tubus (4) to said operating position within said OLT (3)
b. a guide wire substantially accommodated along the length of said OLT; said guide wire is located in an inner canal of said laryngeal mask
wherein said guide wire is adapted for affixing and stabilizing said laryngeal mask within patient's throat.

3. The integrated tubus and laryngeal mask device (1) according to claim 1, wherein one of the following is being held true
a. said OLT (3) can be easily peeled off by using said guide wire
b. said inflating means (8A, 8B) is a pilot balloon valve connected to an inflation lumen extending along said OLT (3) and said tubus (4)
c. said lateral movement preventing element (9, 10) is an O-ring
d. said lateral movement preventing element (9, 10) is a gasket which has the structure and characteristic elements enabling said lateral movement preventing element to be placed between said tubus and said OLT
e. said sealing structure is selected from the group consisting of: a peripheral seal, a radial seal and any combination thereof.

4. The integrated tubus and laryngeal mask device (1) according to claim 1, wherein said lateral movement preventing element (9, 10) is an O-ring.

5. The integrated tubus and laryngeal mask device (1) according to claim 4, wherein one of the following is being held true
a. the sizes of said O-ring are specified by the outside diameter of the tubus (4) and the cross sectional diameter between said tubus (4) and said OLT (3)
b. said O-ring is an elastomeric O-ring for sealing.

6. The integrated tubus and laryngeal mask device (1) according to claim 2, wherein said stopper (2) prevents travel of said tubus (4) lengthwise in said OLT (3).

7. The integrated tubus and laryngeal mask device (1) according to claim 1, additionally comprising a maneuvering mechanism adapted to reorient the distal end of said tubus.

8. The integrated tubus and laryngeal mask device according to claim 1, wherein one of the following is being held true
a. said OLT (3) is further adapted for guiding a working tool to an operating position to view vocal cords and trachea of said patient;
b. said OLT (3) guides a working tool in a manner which allows ventilation through said tubus to continue without interruption.

9. The integrated tubus and laryngeal mask device (1) according to claim 1, additionally comprising a second inflatable balloon (6) located at the distal end of said tubus (4).

10. The integrated tubus and laryngeal mask device (1) according to claim 7, wherein one of the following is being held true
a. said integrated tubus and laryngeal mask device (1) additionally comprising a second valve (7A, 7B) for controlling inflation and deflation of said second balloon (6);
b. said second balloon (6) is made of a flexible material;
c. said second balloon (6) is in physical contact with one side of said tubus (4), whereby upon inflation of said second balloon (6) while said integrated tubus and laryngeal mask device (1) is in place, at least part of said tubus (4) is positioned in a predetermined direction; further wherein said second state is a state in which the internal cavity within said OLT is substantially open.
d. said second inflatable balloon (6) is disposed externally to said flexible airway;
e. said second inflatable balloon (6) is disposed within said flexible airway;
f. said second inflatable balloon (6) is integrated within said flexible airway.

11. The integrated tubus and laryngeal mask device (1) according to claim 1, wherein said balloon cuff (5) is inflated to its first state when the laryngeal mask is inserted, such that it blocks entry into the esophagus of the TOLT.

12. The integrated tubus and laryngeal mask device (1) according to claim 10, wherein said first state is a state of said tubus (4) which prevents entry of the esophagus into the internal cavity within said OLT (3).

13. The integrated tubus and laryngeal mask device (1) according to claim 1, wherein said second inflatable balloon (6) is disposed about one side of, and in physical contact with, said tubus (4).

14. The integrated tubus and laryngeal mask device (1) according to claim 12, wherein said predetermined direction is in the direction of the trachea of said patient.

15. The integrated tubus and laryngeal mask device (1) according to claim 1, wherein one of the following is being held true
a. said OLT (3) has flexible walls integral with the balloon cuff (5) of said OLT and the two are in contact when said balloon cuff is deflated;
b. said balloon cuff (5) is an expansible member made of an elastic material;
c. said balloon cuff (5) is attached to said OLT (3) and communicates with an inflation lumen extending along said OLT and said tubus;
d. said OLT (3) is fixedly secured to said operating position of said tubus (4).

16. The integrated tubus and laryngeal mask device (1) according to claim 1, wherein said balloon cuff (5) is deflated to its second state once said tubus (4) is inserted to the correct position, such that it provides no substantial obstruction within said OLT (3).

## Patentansprüche

1. Integrierte (i) Tubus und (ii) Kehlkopfmaskenvorrichtung (1), umfassend:
a. eine äußere Larynxmaskenröhre (OLT) (3) mit einem proximalen Abschnitt (12) und einem distalen Abschnitt (11); bestehend aus:
i. einer Ballonmanschette (5), die als Kehlkopfmaske geformt ist und mindestens eine aufgeblasene Konfiguration und mindestens eine entleerte Konfiguration aufweist, die in dem distalen Abschnitt (11) angeordnet ist; der Ballon in einer Fluidverbindung mit einer Aufblaseinrichtung (8A, 8B), die an dem proximalen Ende angeordnet ist; und,
ii. einer länglichen Röhre in Verbindung mit der Ballonmanschette (TOLT);
b. eine innere Röhre (Tubus) (4), die innerhalb der OLT (3) angeordnet ist und eine Belüftung durch den Tubus ermöglicht; der Tubus (4) ist durch eine Hauptlängsachse gekennzeichnet; und,
c. mindestens ein Element (9, 10) zur Verhinderung einer seitlichen Bewegung, das in Umfangsrichtung zu dem Tubus (4) und zwischen dem Tubus (4) und der OLT (3) angeordnet ist; adaptiert sowohl (i) um eine seitliche Bewegung des Tubus in Bezug auf die OLT zu verhindern, so dass eine Kontamination der Umgebung zwischen dem Tubus und der OLT verhindert wird; und (ii) um eine longitudinale Hin- und Her Bewegung des Tubus entlang der Hauptlängsachse in Bezug auf die OLT zu ermöglichen, in einer Weise, dass eine Abdichtung in dem inneren Volumen zwischen der OLT und dem Tubus vorgesehen ist wobei, wenn sich die Ballonmanschette (5) in der nicht aufgeblasenen Konfiguration befindet, die Dicke derselben verringert ist, so dass die Ballonmanschette (5) nicht von der TOLT vorsteht.

2. Integrierte Tubus und Kehlkopfmaskenvorrichtung (1) nach Anspruch 1, zusätzlich umfassend ein Element ausgewählt aus einer Gruppe bestehend aus
a. einem Stopper (2), der angepasst ist, um den Tubus (4) fest an der Betriebsposition innerhalb der OLT (3) zu befestigen
b. einem Führungsdraht, der im Wesentlichen entlang der Länge der OLT untergebracht ist; der Führungsdraht befindet sich in einem Innenkanal der Larynxmaske
wobei der Führungsdraht angepasst ist zum Befestigen und Stabilisieren der Kehlkopfmaske innerhalb des Rachens des Patienten.

3. Integrierte Tubus und Kehlkopfmaskenvorrichtung (1) nach Anspruch 1, **dadurch gekennzeichnet, dass** eine der folgenden Aussagen eingehalten wird
a. die OLT (3) kann unter Verwendung des Führungsdrahts leicht abgezogen werden
b. das Aufblasmittel (8A, 8B) ist ein Pilotballonventil, das mit einem Inflationslumen verbunden ist, das sich entlang der OLT (3) und des Tubus (4) erstreckt.
c. das Seitenbewegungsverhinderungselement (9, 10) ist ein O-Ring
d. das Seitenbewegungsverhinderungselement (9, 10) ist eine Dichtung, die die Struktur und die charakteristischen Elemente aufweist, die es ermöglichen, dass das Seitenbewegungsverhinderungselement zwischen dem Tubus und dem OLT platziert werden kann
e. die Dichtungsstruktur ist ausgewählt aus der Gruppe bestehend aus: einer Umfangsdichtung, einer Radialdichtung und einer beliebigen Kombination davon.

4. Integrierter Tubus und Kehlkopfmaskenvorrichtung (1) nach Anspruch 1, wobei das Seitenbewegungsverhinderungselement (9, 10) ein O-Ring ist.

5. Integrierte Tubus- und Kehlkopfmaskenvorrichtung (1) nach Anspruch 4, wobei einer der folgenden Punkte erfüllt ist
a. die Größen des O-Rings sind durch den Außendurchmesser des Tubus (4) und den Querschnittsdurchmesser zwischen dem Tubus (4) und der OLT (3) festgelegt
b. der O-Ring ist ein Elastomer O-Ring zum Abdichten.

6. Integrierter Tubus und Kehlkopfmaskenvorrichtung (1) nach Anspruch 2, wobei der Stopper (2) die Bewegung des Tubus (4) in Längsrichtung in der OLT (3) verhindert.

7. Integrierte Tubus und Kehlkopfmaskenvorrichtung (1) nach Anspruch 1, zusätzlich umfassend einen Manövriermechanismus, der angepasst ist, um das distale Ende des Tubus neu auszurichten.

8. Integrierte Tubus und Kehlkopfmaskenvorrichtung nach einem der Ansprüche 1 bis 7, **dadurch gekennzeichnet, dass** einer der folgenden Punkte erfüllt ist
a. die OLT (3) ist ferner angepasst, um ein Arbeitswerkzeug zu einer Betriebsposition zu führen, um Stimmbänder und Luftröhre des Patienten zu betrachten;
b. die OLT (3) führt ein Arbeitsgerät in einer Weise, die eine ununterbrochene Belüftung durch den Tubus ermöglicht.

9. Integrierte Tubus und Kehlkopfmaskenvorrichtung (1) nach Anspruch 1, zusätzlich umfassend einen zweiten aufblasbaren Ballon (6), der am distalen Ende des Tubus (4) angeordnet ist.

10. Integrierte Tubus und Kehlkopfmaskenvorrichtung (1) nach Anspruch 7, wobei eines der folgenden Gesichtspunkte erfüllt ist
a. wobei die integrierte Tubus und Kehlkopfmaskenvorrichtung (1) zusätzlich ein zweites Ventil (7A, 7B) zum Steuern des Aufblasens und Entleerens des zweiten Ballons (6) aufweist;
b. der zweite Ballon (6) aus einem flexiblen Material hergestellt ist;
c. der zweite Ballon (6) in physischem Kontakt mit einer Seite des Tubus (4) steht, wobei beim Aufblasen des zweiten Ballons (6), während die integrierte Tubus- und Kehlkopfmaskenvorrichtung (1) an Ort und Stelle ist, mindestens ein Teil des Tubus (4) ist in einer vorbestimmten Richtung positioniert; wobei der zweite Zustand ein Zustand ist, in dem der innere Hohlraum innerhalb der OLT im Wesentlichen offen ist;
d. der zweite aufblasbare Ballon (6) außerhalb des flexiblen Luftwegs angeordnet ist;
e. der zweite aufblasbare Ballon (6) innerhalb des flexiblen Luftwegs angeordnet ist;
f. der zweite aufblasbare Ballon (6) ist in den flexiblen Luftweg integriert.

11. Integrierter Tubus und Kehlkopfmaskenvorrichtung (1) nach Anspruch 1, wobei die Ballonmanschette (5) in ihren ersten Zustand aufgeblasen wird, wenn die Kehlkopfmaske eingeführt wird, so dass sie den Eintritt in die Speiseröhre der TOLT blockiert.

12. Integrierte Tubus und Kehlkopfmaskenvorrichtung (1) nach Anspruch 10, wobei der erste Zustand ein Zustand des Tubus (4) ist, der den Eintritt des Ösophagus in den inneren Hohlraum innerhalb der OLT (3) verhindert.

13. Integrierte Tubus und Kehlkopfmaskenvorrichtung (1) nach Anspruch 1, wobei der zweite aufblasbare Ballon (6) um eine Seite des Tubus (4) angeordnet ist und sich in physischem Kontakt mit diesem befindet.

14. Integrierter Tubus und Kehlkopfmaskenvorrichtung (1) nach Anspruch 12, wobei die vorbestimmte Richtung in Richtung der Luftröhre des Patienten ist.

15. Integrierte Tubus und Kehlkopfmaskenvorrichtung (1) nach Anspruch 1, **dadurch gekennzeichnet, dass** eine der folgenden Aussagen eingehalten wird
a. die OLT (3) hat flexible Wände, die integral mit der Ballonmanschette (5) der OLT sind, und die beiden sind in Kontakt, wenn die Ballonmanschette entleert ist;
b. die Ballonmanschette (5) ist ein dehnbares Element, das aus einem elastischen Material hergestellt ist;
c. die Ballonmanschette (5) ist an der OLT (3) angebracht und kommuniziert mit einem Inflationslumen, das sich entlang der OLT und des Tubus erstreckt;
d. die OLT (3) ist fest an der Betriebsposition des Tubus (4) befestigt.

16. Integrierter Tubus und Kehlkopfmaskenvorrichtung (1) nach Anspruch 1, wobei die Ballonmanschette (5) in ihren zweiten Zustand entleert wird, sobald der Tubus (4) in die korrekte Position eingeführt ist, so dass er keine wesentliche Behinderung bereitstellt innerhalb der OLT (3).

## Revendications

1. Un dispositif de masque (1) tubulaire (i) et laryngé (ii) intégré, comprenant:
a. un tube de masque laryngé externe (OLT) (3) ayant une partie proximale (12) et une partie distale (11); comprenant:
i. un ballonnet manchette (5) en forme de masque laryngé, ayant au moins une configuration gonflée et au moins une configuration dégonflée, située dans ladite partie distale (11); ledit ballonnet étant dans une connexion fluidique avec un moyen de gonflage (8A, 8B) situé à ladite extrémité proximale; et,
ii. un tube allongé en liaison avec ledit ballonnet (TOLT);
b. un tube interne (tube) (4) situé à l'intérieur dudit OLT (3) et permettant une ventilation à travers le tube; ledit tube (4) est **caractérisé par** un axe longitudinal principal; et,
c. au moins un élément empêchant un mouvement latéral (9, 10), situé circonférentiellement par rapport audit tube (4) et entre ledit tube (4) et ledit OLT (3); adapté à la fois (i) pour empêcher le mouvement latéral dudit tube par rapport audit OLT, de manière à empêcher la contamination de l'environnement entre ledit tube et ledit OLT; et (ii) permettre un mouvement de va-et-vient longitudinal dudit tube le long dudit axe longitudinal principal par rapport audit OLT, de manière à assurer l'étanchéité dans le volume intérieur entre le OLT et le tube,
dans lequel, lorsque ledit ballonnet manchette (5) est dans ladite configuration dégonflée, son épaisseur est réduite de telle sorte que ledit ballonnet manchette (5) ne fait pas saillie par rapport audit TOLT.

2. Dispositif de masque tubaire et laryngé intégré (1) selon la revendication 1, comprenant, en outre, un élément choisi dans un groupe comprenant
a. un bouchon (2) adapté pour fixer de manière fixe ledit tube (4) à ladite position de fonctionnement à l'intérieur dudit OLT (3)
b. un fil de guidage logé sensiblement sur la longueur dudit OLT; ledit fil de guidage est situé dans un canal interne dudit masque laryngé
dans lequel ledit fil de guidage est adapté pour fixer et stabiliser ledit masque laryngé dans la gorge du patient.

3. Dispositif de masque tubaire et laryngé intégré (1) selon la revendication 1, dans lequel l'un des éléments suivants est respecté
a. ledit OLT (3) peut être facilement décollé en utilisant ledit fil de guidage
b. ledit moyen de gonflage (8A, 8B) est une valve à ballonnet pilote connectée à une lumière de gonflage s'étendant le long dudit OLT (3) et dudit tube (4)
c. ledit élément empêchant le mouvement latéral (9, 10) est un joint torique
d. ledit élément empêchant le mouvement latéral (9, 10) est un joint d'étanchéité qui a la structure et des éléments caractéristiques permettant le placement dudit élément empêchant le mouvement latéral entre ledit tube et ledit OLT
e. ladite structure d'étanchéité est choisie dans le groupe comprenant: un joint d'étanchéité périphérique, un joint d'étanchéité radial et toute combinaison de ceux-ci.

4. Dispositif de masque tubaire et laryngé intégré (1) selon la revendication 1, dans lequel ledit élément empêchant le mouvement latéral (9, 10) est un joint torique.

5. Dispositif de masque tubaire et laryngé intégré (1) selon la revendication 4, dans lequel l'un des éléments suivants est respecté
a. les dimensions dudit joint torique sont spécifiées par le diamètre extérieur du tube (4) et le diamètre de la section transversale entre ledit tube (4) et ledit OLT (3)
b. ledit joint torique est un joint torique en élastomère pour l'étanchéité.

6. Dispositif de masque tubulaire et laryngé intégré (1) selon la revendication 2, dans lequel ledit bouchon (2) empêche le déplacement dudit tube (4) dans le sens de la longueur dans ledit OLT (3).

7. Dispositif de masque tubaire et laryngé intégré (1) selon la revendication 1, comprenant, en outre, un mécanisme de manoeuvre apte à réorienter l'extrémité distale dudit tube.

8. Dispositif de masque tubaire et laryngé intégré (1) selon l'une des revendications précédentes, **caractérisé en ce que** l'un des éléments suivants est respecté
a. ledit OLT (3) est en outre adapté pour guider un outil de travail dans une position de travail afin de visualiser les cordes vocales et la trachée dudit patient;
b. ledit OLT (3) guide un outil de travail de manière à permettre à la ventilation à travers ledit tube de continuer sans interruption.

9. Dispositif de masque tubaire et laryngé intégré (1) selon la revendication 1, comprenant, en outre, un deuxième ballonnet gonflable (6) situé à l'extrémité distale dudit tube (4).

10. Dispositif de masque tubaire et laryngé intégré (1) selon la revendication 7, dans lequel l'un des éléments suivants est respecté.
a. ledit dispositif de masque tubaire et laryngé intégré (1) comprenant, en outre, une seconde valve (7A, 7B) pour contrôler le gonflage et le dégonflage dudit second ballonnet (6);
b. ledit second ballonnet (6) est constitué d'un matériau flexible;
c. ledit second ballonnet (6) est en contact physique avec un côté dudit tube (4), de sorte que lors du gonflage dudit second ballonnet (6) pendant que ledit dispositif intégré de masque tubaire et laryngé (1) est en place, au moins une partie dudit tube (4) est positionné dans une direction prédéterminée; dans lequel ledit second état est un état dans lequel la cavité interne à l'intérieur dudit OLT est sensiblement ouverte ;
d. ledit second ballonnet gonflable (6) est disposé à l'extérieur de ladite voie respiratoire flexible;
e. ledit second ballonnet gonflable (6) est disposé à l'intérieur de ladite voie respiratoire flexible;
f. ledit second ballonnet gonflable (6) est intégré à l'intérieur de ladite voie respiratoire flexible.

11. Dispositif de masque tubaire et laryngé intégré (1) selon la revendication 1, dans lequel ledit ballonnet manchette (5) est gonflé à son premier état lorsque le masque laryngé est inséré, de sorte qu'il bloque l'entrée dans l'oesophage du TOLT.

12. Dispositif de masque tubaire et laryngé intégré (1) selon la revendication 10, dans lequel ledit premier état est un état dudit tube (4) qui empêche l'entrée de l'oesophage dans la cavité interne à l'intérieur dudit OLT (3).

13. Dispositif de masque tubaire et laryngé intégré (1) selon la revendication 1, dans lequel ledit second ballon gonflable (6) est disposé autour d'un côté dudit tube (4) et en contact physique avec celui-ci.

14. Dispositif de masque tubaire et laryngé intégré (1) selon la revendication 12, dans lequel ladite direction prédéterminée est dans la direction de la trachée dudit patient.

15. Dispositif de masque tubaire et laryngé intégré (1) selon la revendication 1, dans lequel l'un des éléments suivants est respecté
a. ledit OLT (3) a des parois flexibles solidaires du ballonnet manchette (5) dudit OLT et les deux sont en contact lorsque ledit ballonnet est dégonflé;
b. ledit ballonnet manchette (5) est un élément extensible constitué d'un matériau élastique;
c. ledit ballonnet manchette (5) est attaché audit OLT (3) et communique avec une lumière de gonflage s'étendant le long dudit OLT et dudit tube;
d. ledit OLT (3) est fixé solidement à ladite position de fonctionnement dudit tube (4).

16. Dispositif de masque tubaire et laryngé intégré (1) selon la revendication 1, dans lequel ledit ballonnet manchette (5) est dégonflé dans son deuxième état une fois que ledit tube (4) est inséré dans la position correcte, de sorte qu'il ne procure pas d'obstruction substantielle dans ladite terminaison OLT (3).
